# EUROPEAN PATENT SPECIFICATION

(11) **EP 0 487 218 B1**
(45) Date of publication and mention of the grant of the patent: **29.12.1997**
(21) Application number: 91310062.4
(22) Date of filing: 31.10.1991
(51) Int. Cl.: C12Q 1/68

(54) **Method for detecting or quantifying target nucleic acid**
Verfahren zum Nachweis oder Quantifizierung von Zielnukleinsäuren
Procédé pour la détection ou détermination quantitative d'acides nucléiques cibles

(30) Priority: 31.10.1990 JP 294305/90
(43) Date of publication of application: 27.05.1992
(73) Proprietor: Tosoh Corporation, Shinnanyo-shi, Yamaguchi-ken, 746 (JP)
(72) Inventor: Mitoma, Yasutami, Fujisawa-shi, Kanagawa 252 (JP)
(74) Representative: Kearney, Kevin David Nicholas

(56) References cited:
- EP-A- 0 200 362
- WO-A-90/15881
- BE-A- 1 000 572
- US-A- 4 257 774
- CLINICAL CHEMISTRY vol. 35, no. 9, September 1989, WINSTON US pages 1826 - 1831; H. LOMELI ET AL.: 'QUANTITATIVE ASSAYS BASED ON THE USE OF REPLICATABLE HYBRIDIZATION PROBES'
- DNA replication, Kornberg 1980, pages 427-434
- DNA replication, Kornberg & Baker 1992, pages 451-456
- J.Mol.Biol, 1974, vol.89, pages 719-736

## Description

### I. Field of the Invention

The present invention relates to a method of detecting or quantifying a target nucleic acid in a sample.

### II. Description of the Related Art

In the conventional clinical tests of infectious diseases caused by viruses and bacteria, biological samples such as tissues, body fluids and excretions are cultured and the existence of the viruses and bacteria is checked. However, culturing of the samples is time-consuming and sometimes technically difficult. Further, the operator may be infected with the pathogens during the culturing of the samples.

As the methods for quickly identifying the pathogens, methods for detecting antigens by utilizing antigen-antibody reaction, and methods for detecting nucleic acid by using DNA probes are employed in practice. However, with the antigen-detecting methods utilizing the antigen-antibody reactions, the pathogens may not be detected when the antigens are concealed. Further, since a bacterium has a large number of antigens, it is not easy to discover a specific antigen which is unique to the bacterium of interest. Still further, a certain number or density of bacterial cells or viruses is needed for the detection, so that if the number of the bacterial cells or the viruses are smaller than the detection limit, the pathogen cannot be detected. Thus, the immunological detection methods are not satisfactcry with respect to the sensitivity of the detection.

On the other hand, in the methods for detecting nucleic acids by using DNA probes, it is relatively easy to find a specific nucleic acid region which is unique to a bacterium or a virus. By cloning or synthesizing the whole or a part of this specific region, a nucleic acid which can be used as a DNA probe is prepared. Recently, a method for amplifying a nucleic acid called polymerase chain reaction was retorted (Saiki et al., SCIENCE, 230, 1350-1354, 1985). By the polymerase chain reaction, a region in a single nucleic acid molecule can be amplified to as much as 1,000,000-fold in a test tube. By utilizing the polymerase chain reaction, a particular region of a target nucleic acid can be detected after amplification thereof, so that a pathogen may be detected from one molecule of the nucleic acid. After the polymerase chain reaction, the amplified region is detected by subjecting the reaction mixture to agarose gel electrophoresis and staining the bands, or by subjecting the reaction mixture to the dot hybridization method.

However, with the method of detecting nucleic acids utilizing the polymerase chain reaction, the reaction mixture after the polymerase chain reaction is removed from the reaction vessel and is subjected to the assay. Thus, the amplified nucleic acid region may contaminate other samples in the laboratory. If such a contamination occurs, the contaminant serves as a template in the contaminated sample, so that a false positive result may be obtained. This is recognized as a large problem in the detection methods of nucleic acids utilizing the polymerase chain reaction. Further, the detection methods utilizing the polymerase chain reaction also has a drawback in that it is difficult to determine the initial concentration of the target nucleic acid.

WO-A-9015881 discloses a method of detecting nucleic acid sequences in biological samples in which the nucleic acid is amplified by an appropriate amplification technique amongst which PCR is mentioned. This can be carried out in a sealed vessel. Subsequently, the amplified sample is removed from the vessel and treated by electrophoresis. The target nucleic acid is detected by use of a fluorescent dye such as ethidium bromide. The intercalating pigment is always only added to the nucleic acid after the replication procedure has been completed.

EP-A-0200362 discloses the basic method of PCR. It does not disclose or suggest the detection of a target nucleic acid by using a fluorescent pigment. In this disclosure, after amplifying a target nucleic acid by PCR, the amplified nucleic acid is detected by the Southern blotting method or the like.

H. Lomeli *et al, Clinical Chemistry **35,*** No 9: 1826-1831 (September 1989) disclose a procedure which comprises the steps of capturing the target nucleic acid in a sample by capture probes immobilised on paramagnetic particles so as to separate the target nucleic acid; reacting the captured target nucleic acid with RNA probes (replicatable probes) having a region replicated by Qβ replicase and a region complementary with the target nucleic acid so as to form a complex therebetween (see the drawing on page 1828, upper right portion); removing the RNA probes which did not form the complex; adding Qβ replicase to the complex so as to replicate the region in the RNA probe, which can be replicated by Qβ replicase; and finally detecting the single-stranded RNA replicated by Qβ nucleic acid.

### SUMMARY OF THE INVENTION

Accordingly, an object of the present invention is to provide a method for detecting a target nucleic acid utilizing the polymerase chain reaction, with which the problem of the contamination can be avoided.

Another object of the present invention is to provide a method for quantifying a target nucleic acid utilizing the polymerase chain reaction, by which the initial concentration of the target nucleic acid can be determined and with which the problem of the contamination can be avoided.

According to the present invention, a method for detecting a target nucleic acid in a sample comprises:
amplifying a region of said target nucleic acid;
contacting or treating the amplified region with a fluorescent pigment of which the fluorescent property is changed upon reaction with the nucleic acid; and
detecting or measuring the change in the said fluorescence of the said marker, characterised in that the fluorescent pigment is added to the original sample and located in a reaction vessel, and amplification and detecting or measuring the fluorescence is carried out in the reaction vessel in the presence of the fluorescent pigment and in that the amplification is carried out by polymerase chain reaction.

Measuring the fluorescence intensity of the fluorescent pigment may be carried out before and after the polymerase chain reaction, or during polymerase chain reaction.

The invention also extends to a kit for detecting or quantifying a target nucleic acid in a sample, comprising:
(A) a fluorescent pigment, the fluorescence of which changes upon reaction with a double-stranded nucleic acid; and
(B) reagents for amplifying said target nucleic acid by the polymerase chain reaction (PCR),
wherein the said fluorescent pigment and the said reagents are contained in a single vessel.

The said fluorescent pigment is preferably one of which the fluorescence changes upon intercalation into the said double-stranded nucleic acid.

The said fluorescent pigment is preferably water-soluble and thermally stable at temperatures at which the said PCR is conducted.

The said vessel is preferably provided with sealing means.

Intercalating fluorescent pigments are known to have a property that the fluorescent property thereof is drastically changed when they are intercalated into double-stranded nucleic acids. In the method of the present invention, such a fluorescent pigment is employed. The reaction mixture before the polymerase chain reaction (hereinafter also referred to as "PCR" for short) contains a very small amount of a nucleic acid acting as a template, at least one pair of DNA primers, a polymerase and mononucleoside triphosphates, and the fluorescent pigment gives a certain fluorescent property in the presence of these components. In cases where the sample does not contain the target nucleic acid to be detected or quantified, since the DNA in the reaction mixture is not amplified, the fluorescent property of the pigment does not substantially change before and after PCR. In contrast, if a target nucleic acid to be detected or quantified is contained in the sample, the target nucleic acid is amplified by PCR and the fluorescent property of the pigment is changed since the pigment reacts with or performs differently in the presence of or an exposure to the amplified nucleic acid. Thus, by checking the fluorescence property of the pigment before and after PCR, the target nucleic acid can be detected. Further, if the initial concentration of the target nucleic acid is high, the change in the fluorescent property occurs after a small number of repetitions of the PCR cycles. In the contrast, if the initial concentration of the target nucleic acid is low, a larger number of PCR cycles are required for the pigment to change its fluorescent property. Thus, by monitoring the fluorescence intensity during the PCR, if calibration curves are preliminarily prepared by using standard samples containing known amounts of the target nucleic acid, the initial concentration of the target nucleic acid contained in the sample can be determined.

According to the method of the present invention, since the measurement of the fluorescence intensity of the pigment can be carried out without removing the reaction mixture after the PCR from the reaction vessel, the possibility of the contamination of other samples with the amplified nucleic acid can be largely reduced. In a preferred embodiment wherein all steps are carried out within a sealed vessel, the possibility of the contamination can be substantially eliminated. Further, by the method of the present invention, the initial concentration of the target nucleic acid can be determined. Thus, by the present invention, the drawback of the conventional PCR-based detection method that the initial concentration of the target nucleic acid cannot be determined can be overcome. Further, since the method of the present invention can be carried out by merely adding the fluorescent pigment to the reaction mixture of the conventional PCR process, the method is simple and so does not require skillfulness. Thus, a large number of samples can be processed, so that the method of the present invention can be applied to screening methods utilizing PCR. Still further, whether the amplification occurred or not can be judged based on the measured fluorescence intensity, the detection of quantification method can be automated. Thus, the present invention will greatly contribute to the field of diagnosis.

### BRIEF DESCRIPTION OF THE DRAWINGS

Fig. 1 schematically shows an electrophoresis pattern of a reaction mixture after PCR;
Fig. 2 shows the relationship between the number of cycles of PCR and the relative fluorescence intensity; and
Fig. 3 shows the relationship between the number of cycles of PCR and the relative fluorescence intensity, wherein three kinds of samples containing different concentrations of the template DNA are tested.

### DETAILED DESCRIPTION OF THE PREFERRED EMBODIMENTS

The target nucleic substance to be detected or quantified may be of any origin of interest, such as bacteria, viruses or tissues.

In the method according to a first aspect of the present invention, a region of the target nucleic acid is amplified by the PCR. This step can be carried out conventionally as described by Saiki et al (supra). That is, a cycle of denaturation of the target nucleic acid, annealing of the target nucleic acid and a pair of DNA primers and extension of complementary chains by the action of a polymerase such as Tag DNA polymerase is repeated, for example, 30 times. This step per se is known and described more concretely in the Examples hereinbelow described.

After the PCR step, a fluorescent pigment of which fluorescent property is changed upon reaction with nucleic acid is added to the reaction mixture so as to allow the reaction thereof with the amplified target nucleic acid.

As the fluorescent pigment having such a property, intercalating fluorescent pigments are preferred. Intercalating fluorescent pigments are known to intercalate between bases of nucleic acids and their fluorescence property is largely changed upon intercalation in the nucleic acid. Since the reaction mixture is aqueous and contains a protein, those pigments which are water-soluble and are unlikely to be affected by proteins are preferred. Further, since the PCR requires temperature changes from room temperature to a high temperature, those which are thermally stable are preferred. Examples of the fluorescent pigments satisfying these preferred properties include the following:
(1) ethidium bromide and derivatives thereof (e.g., those described in Biochemistry 27, 7919 (1988); J. Cell Biol., 59, 766 (1973); Biochemistry 17, 5078 (1978) and Biochemistry 22, 3231 (1983));
(2) acridine orange and derivatives thereof (e.g., those described in Cytometry 9, 325 (1988); Proc. Natl Acad. Sci. 72, 2915 (1975); and Biochemistry 17, 5071 (1978));
(3) bis-bentimide and derivatives thereof (e.g., those described in Exp. Cell Res. 174, 388 (1988); Cold Harbor Symposia on Quant. Biol. 51, 151 (1986); Nucleic Acids Res. 15, 10589 (1987));
(4) diaminophenylindole and derivatives thereof (e.g., those described in Plant Sci. 55, 151 (1988); Proc. Natl. Acad. Sci. 83, 2934 (1986));
(5) actinomicins and derivatives thereof (e.g., those described in Cytometry 1, 2 (1980); Histochem. 72, 11 (1981));
(6) thiazole orange and derivatives thereof (e.g., those described in Cytometry 7, 508 (1986); Cyotometry 8, 568 (1987); Nippon-Kanko-shikiso Catalogue NK-321 NK-731 and the like, and
(7) chromomycins and derivatives thereof (e.g., those described in Antibiot. Chemoth. 12, 182 (1962); Anticancer Research, 1, 341 (1981))

### These substances are well-known in the art.

In the method of the present invention, in cases where an intercalating fluorescent pigment is employed, the phenomenon that the fluorescent property is changed between the pigment which is free in the aqueous solution and the pigment which is intercalated in double-stranded nucleic acids is utilized as mentioned above. This change in the fluorescent property can be detected as a change in its fluorescence intensity, although the change in the fluorescence intensity may stem from the change in the fluorescence spectrum. By comparing the peak wavelengths of the excitation light and the emitted light in the presence and absence of the double-stranded nucleic acid, the intercalating fluorescent pigments may be grouped into the following four groups:
1) Both of the peak wavelengths of the excitation light and the emitted light do not change, i.e. the peak wavelength of the excitation light is kept the same, (in this case, only the fluorescence intensity is changed);
2) The peak wavelength of the excitation light is shifted while that of the emitted liht does not change, i.e. the peak wavelength of the excitation light is changed by the operator, but the emitted light stays the same; =
3) The peak wavelength of the excitation light is not changed, i.e. is kept the same, while that of the emitted light is shifted; and
4) Both of the peak wavelengths of the excitation light and the emitted light are shifted, i.e. the peak wavelength of the excitation light is changed by the operator and the emitted light also changes.
These changes in the peak wavelength may accompany change in the fluorescence intensity at the peak wavelengths. For example, acridine orange and derivatives thereof may be regarded as belonging to the group 1) even though the peak wavelength of the excitation light is shifted by several nanometers. Bis-bentimide and derivatives thereof (e.g., Hoechst 33258 employed in the examples later described) may be regarded as belonging to the group 3). Most of the intercalating fluorescent pigments are grouped into group 4) (Strictly speaking, acridine orange and Hoechst 33258 are also grouped in this group). Thus, by measuring the fluorescent spectrum of the pigment before and after or during the PCR process, the amplification of the nucleic acid may be detected (even in this case, fluorescence intensity is measured for measuring the fluorescent spectrum). However, the amplification of the nucleic acid may be conveniently detected by measuring the fluorescence intensity of the emitted light under the optimum conditions of the pigment in the presence of the double-stranded DNA (i.e., the pigment is excited with an excitation light having a wavelength in the vicinity of the peak wavelength of the excitation light and the emitted light is measured at a wavelength in the vicinity of the peak wavelength of the emitted light)

The amount of the fluorescent pigment to be added to the reaction mixture may preferably be selected such that the pigment is attached to (intercalated in) the amplified nucleic acid in the amount of one molecule per several to 100 bases of the amplified nucleic acid.

The reaction between the fluorescent pigment and the amplified target nucleic acid may be carried out at any temperature between 0 - 100°C, but for economy, the reaction may preferably and conveniently be carried out at room temperature. The reaction is usually completed in a relatively short time, for example, 10 seconds to 3 minutes.

The fluorescence intensity of the pigment can be measured by a conventional method well-known in the art. It is preferred to measure the fluorescence quantitatively by using a fluorescence spectrophotomater. However, in cases where only qualititative detection is desired, the existence of the target nucleic acid may be judged by observing the reaction mixture with naked eyes or a fluorescence microscope while irradiating thereto an excitation light or by taking a fluorescence photograph. In order to prevent contamination, the measurement of the fluorescence is carried out without removing the reaction mixture, so that the reaction vessel is preferably made of a material which allows for the penetration of the excitation light, which is thermally stable and which hardly adsorbs proteins. Preferred examples of the materials satisfying these requirements include polypropylene resins, silicone-coated glass, polymethyl pentene resins and the like. By employing such materials, the reaction vessel may be applied to the fluorescence meter.

For promoting the accuracy of the method, it is preferred to carry out a negative control experiment using a sample containing no target nucleic acid, although this is not essential.

In the method according to a second aspect of the present invention, the fluorescent pigment is contained in the reaction mixture before the PCR process. In this method, the fluorescent pigment and the amount thereof to be added to the reaction mixture is the same as described before. Since the reaction between the pigment and the amplified target nucleic acid proceeds during the PCR process, the measurement of the fluorescence intensity may be carried out immediately after the PCR process. The PCR process and the measurement of the fluorescence can be carried out in the same manner as described above. The fluorescent pigment may be added to the reaction mixture during the PCR process. That is, the PCR process may be once stopped, the fluorescent pigment may be added to the reaction mixture and then the PCR process may be started again. This mode is also within the scope of the present invention.

According to the second aspect of the present invention, since the fluorescent pigment is contained in the starting mixture, the PCR process and the measurement of the fluorescence intensity can be carried out using a sealed vessel. thus, the possibility of the contamination of other samples with the amplified target nucleic acid can be substantially eliminated.

According to a third aspect of the present invention, the fluorescent pigment is contained in the starting reaction mixture as in the method according to the second aspect of the present invention, and the fluorescence intensity is monitored during the PCR process. Although the monitoring of the fluorescence intensity can be made by taking an aliquote of the reaction mixture and measuring the fluorescence intensity thereof, since the overall process is preferably carried out in a sealed vessel, the starting reaction mixture may be divided into several to ten and several portions, and the each portion may be subjected to the PCR method. By terminating the PCR at different number of repetitions of the PCR cycles for each portion and by measuring the fluorescence intensity for each portion, the monitoring of the fluorescence intensity during the PCR process may be attained. With this mode, the monitoring can be carried out without opening the vessel for removing an aliquote of the sample. In another preferred mode, the PCR is carried out in a single reaction vessel and the fluorescence intensity of the reaction mixture is continuously or intermittently measured by a fluorescence spectrophotometer or the like. As will be shown by experiments in the Examples hereinbelow described, the fluorescence intensity is sharply increased at a certain time point depending on the initial concentration of the target nucleic acid. Thus, by monitoring the fluorescence intensity during the PCR process, the initial concentration can be determined based on calibration curves prepared by using standard samples containing known amounts of the target nucleic acid.

The invention will now be described by way of examples thereof. It should be noted, however, the examples are presented for the illustration purpose only and should not be interpreted in any restrictive way.

### Example 1

Using a PCR kit (GeneAmp (trademark) commercially available from Takara Shuzo Co., Ltd., Kyoto, Japan), the λDNA included in the kit as a standard was amplified using a pair of primers also included in the kit as a standard reagent. To the reaction mixture, Hoechst 33258 pigment commercially available from Hoechst AG, which is an intercalating fluorescent pigment, was added to a final concentration of 1 µg/ml. The starting reaction mixture had the following composition:

| | |
|---|---|
| λDNA (0.1 µg/ml) | 10 µl |
| 10 X Reaction Buffer | 10 µl |
| Hoechst 33258 Pigment (10 µg/ml) | 10 µl |
| Primers 1 and 2 (20 µM) | each 5 µl |
| dNTP Mixture (each 2.5 mM) | 8 µl |
| Water | 51.5 µl |
| Tag Polymerase (5 units/µl) | 0.5 µl |

The above-mentioned 10 X reaction buffer had the following composition:

| | |
|---|---|
| Potassium chloride | 0.5 M |
| Tris-HCl Buffer (pH 8.3) | 0.1 M |
| Magnesium chloride | 15 mM |
| Gelatin | 0.01% (w/v) |

The primers 1 and 2 had the following base sequences:
Primer 1: 7131 - 7155 nucleotide sequence of λDNA (complementary to the - chain) GATGAGTTCGTGTCCGTACAACTGG Primer 2: 7606 - 7630 nucleotide sequence of λDNA (complementary to the ö chain)

GGTTATCGAAATCAGCCACAGCGCC

After the mixture is placed in a reaction vessel, the reaction vessel was sealed and the PCR was carried out under temperature conditions of 94°C for 1 minute (denaturation), 37°C for 1 minute (annealing) and 72°C for 1 minute (extension of DNA chain by Tag DNA polymerase).

After the PCR, the reaction vessel was applied to a fluorescence spectrophotometer (Hitachi 850 type) without opening the seal of the vessel, and the fluorescence intensity from the reaction vessel was measured (excitation light: 343 nm, emission light: 483 nm, PM Gain: High, Response: 2 seconds, EM Filter: 350 nm). Further, as a negative control, the same process was repeated except that the starting reaction mixture did not contain the λDNA.

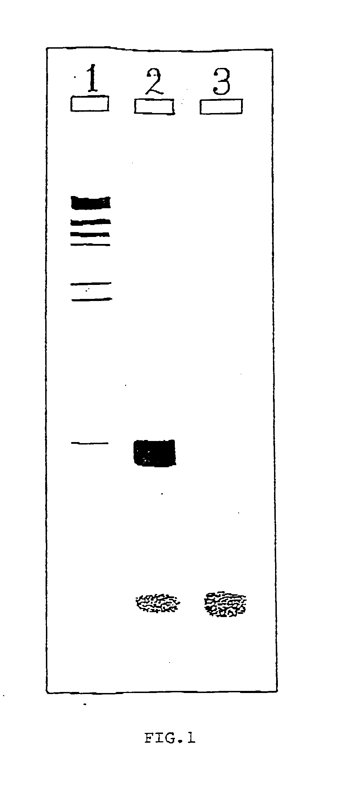

As a result, the fluorescence intensity of the positive experiment was 1260 while that in the negative control was 275, so that a clear difference was observed. An aliquote of the reaction mixture after the PCR was applied to 1% agarose gel containing Tris, boric acid and EDTA and electrophoresis was carried out at room temperature for 30 minutes at 100v to obtain an electrophoretic pattern shown in Fig. 1. In Fig. 1, lane 1 shows the electrophoretic pattern of molecular weight markers, lane 2 shows the electrophoretic pattern of the sample containing the template nucleic acid, which was subjected to the PCR, and lane 3 shows the electrophoretic pattern of the negative control containing no template nucleic acid. From this electrophoretic pattern, it was also confirmed that amplification of the λDNA occurred in the positive experiment.

### Example 2

The same procedure as in Example 1 was repeated except that the fluorescence intensities of the positive and negative samples were measured before and after the PCR. The results are as follows:

| | Fluorescence Intensity | |
|---|---|---|
| | Before PCR | After PCR |
| Negative Experiment | 233 | 270 |
| Positive Experiment | 205 | 1170 |

As can be seen from these results, although an increase in the fluorescence intensity is observed in the negative control, the fluorescence intensity after the PCR is only 1.2 times that before the PCR. In contrast, in the positive experiment, the fluorescence intensity after the PCR is as much as 5.7 times that before the PCR, so that the difference in the increase in the fluorescence intensity was clear.

### Example 3 (Detection of Chromosomal DNA Originating from Bacterium)

Mycobacterium smegmatis was cultured in Dubos'es medium for 5 days, the bacterial cells were collected by centrifugation. An aliquote thereof weighing 100 mg was used as the standard bacterial cells. The standard bacterial cells were suspended in 0.5 ml of a buffer having the composition described below, and then 0.1 ml of 20% dodecyl sodium sulfate was added thereto. To the resulting mixture, an equivolume of phenol/chloroform mixture (1:1 (v/v)) was added and the resultant was vigorously stirred, followed by leaving the resulting mixture to stand at 60°C for 10 minutes. The resulting mixture was centrifuged and the aqueous phase was recovered. To the aqueous phase, 2.5 times volume of ethanol was added and the resultant was left to stand at -80°C for 30 minutes, followed by centrifugation to precipitate nucleic acids. The thus obtained precipitate was washed with 70% ethanol and was dried in vacuum. The thus obtained nucleic acid was dissolved in 100 µl of TE buffer, and this solution was used as the standard DNA. The above-mentioned buffer had the following composition:

| COMPOSITION OF BUFFER | |
|---|---|
| Sodium Chloride | 100 mM |
| Tris-HCl Buffer | 10 mM |
| EDTA | 1 mM |
| | pH 7.5 |

The chromosomal 16S rRNA gene of the standard DNA was amplified by the PCR process as in Example 1. The starting reaction mixture had the following composition:

| | |
|---|---|
| Standard λDNA | 10 µl |
| 10 X Reaction Buffer | 10 µl |
| Hoechst 33258 Pigment (10 µg/ml) | 10 µl |
| Primer 1 | 5 µl |
| Primer 2 | 5 µl |
| dNTP mixture (each 2.5 mM) | 8 µl |
| Water | 60.5 µl |
| Tag Polymerase (5 units/µl) | 0.5 µl |

The primers had the following base sequences:

Primer 1: 5'TAACACATGCAAGTCGAACGG3'
Primer 2: 5'AACTGAGACCGGCTTTTAAGGATT3'

The fluorescence intensity was measured before and after the PCR without opening the seal of the vessel in the same manner as in Example 1.

As a result, the fluorescence intensity before the PCR was 530 while that after the PCR was 902, so that the latter was about 1.7 times the former. Thus, the existence of the target nucleic acid was confirmed through the amplification thereof.

### Example 4

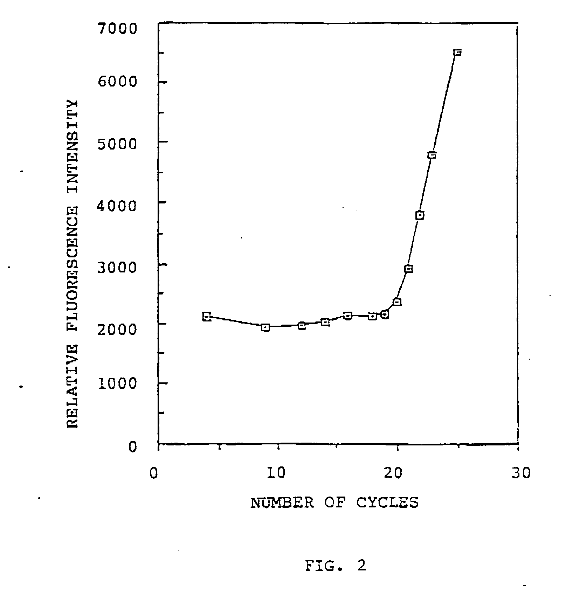

Six hundred microliters of a starting reaction mixture having the same composition as that employed in Example 1 except that the concentration of the λDNA serving as a template was 1/10 that in Example 1, was prepared. The mixture was then equally divided into 12 portions (50 µl each), and each portion was placed in a microtube. The PCR was carried out under temperature conditions of 94°C for 30 seconds (denaturation), 45°C for 20 seconds (annealing) and 72°C for 1 minute (extension of DNA by Tag DNA polymerase). The cycle was repeated 25 times. A tube was removed from the thermal cycler at 0, 9, 12, 14, 16, 18, 19, 20, 21, 22, 23 or 25 cycles, and was set to a holder of a fluorescence detector, which was kept at 72°C, followed by the measurement of fluorescence intensity (excitation light: 350 nm; emission light: 450 nm). Fig. 2 graphically shows the results, wherein the number of the cycles are taken along the abscissa and the measured relative fluorescence intensities are taken along the ordinate. These results indicate that the fluorescence intensity can be monitored during the PCR process under sealed condition. Fig. 2 also indicates that the relative fluorescence intensity is sharply increased at a certain time point.

### Example 5

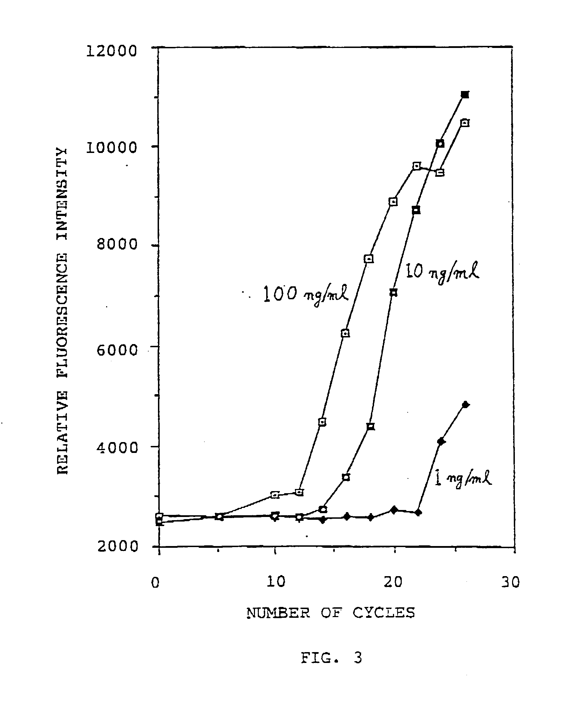

Three kinds of reaction mixtures each of which had a volume of 300 µl containing varying amounts of the template λDNA were prepared. The concentrations of the template DNA in each was 100 ng/ml, 10 ng/ml or 1 ng/ml. The concentrations of the primers were 1/5 that in Example 1. The composition of each mixture was the same as in Example 1 except for the above-mentioned concentrations of the template DNA and the primers. Each of the reaction mixtures were equally divided into 12 portions (25 µl) and each portion was placed in a tube. The PCR was carried out under temperature conditions of 94°C for 1 minute (denaturation), 37°C for 1 minute (annealing) and 72°C for 1 minute (extension of DNA by Tag DNA polymerase). The cycle was repeated 26 times. A tube was removed from the thermal cycler at 0, 5, 10, 12, 14, 16, 18, 20, 22, 24, or 26 cycles, and the fluorescence intensity of each mixture was determined at room temperature as in Example 1. The results are shown in Fig. 3. Fig. 3 clearly shows that the higher the initial concentration of the template DNA, the earlier the sharp increase in the relative fluorescence intensity occurs. Thus, it was confirmed that the initial concentration of the target nucleic acid can be determined by monitoring the fluorescence intensity during the PCR process.
SEQ ID NO: 1
   - SEQUENCE TYPE:: 7131-7155 Nucleotide sequence of λDNA (complementary to the - chain)
   - SEQUENCE LENGTH:: 25 base pairs GATGAGTTCG TGTCCGTACA ACTGG 25
SEQ ID NO: 2
   - SEQUENCE TYPE:: 7606-7630 Nucelotide sequence of λDNA (complementary to the + chain)
   - SEQUENCE LENGTH:: 25 base pairs GGTTATCGAA ATCAGCCACA GCGCC 25
SEQ ID NO: 3
   - SEQUENCE TYPE:: Primer 1 nucelotide sequence
   - SEQUENCE LENGTH:: 21 base pairs 5'TAACACATGC AAGTCGAACG G3' 21
SEQ ID NO: 4
   - SEQUENCE TYPE:: Primer 2 nucleotide sequence
   - SEQUENCE LENGTH:: 24 base pairs

5'AACTGAGACC GGCTTTTAAG GATT3' 24

## Claims

1. A method for detecting a target nucleic acid in a sample, comprising:
amplifying a region of said target nucleic acid;
contacting or treating the amplified region with a fluorescent pigment of which the fluorescent property is changed upon reaction with the nucleic acid; and
detecting or measuring the change in the said fluorescence of the said marker, characterised in that the fluorescent pigment is added to the original sample and located in a reaction vessel and amplification and detecting or measuring the fluorescence is carried out in the reaction vessel in the presence of the fluorescent pigment and in that the amplification is carried out by polymerase chain reaction.

2. A kit for detecting or quantifying a target nucleic acid in a sample, comprising:
(A) a fluorescent pigment, the fluorescence of which changes upon reaction with a double-stranded nucleic acid; and
(B) reagents for amplifying said target nucleic acid by the polymerase chain reaction (PCR),
wherein the said fluorescent pigment and the said reagents are contained in a single vessel.

3. The kit of claim 2, wherein the said fluorescent pigment is one of which the fluorescence changes upon intercalation into the said double-stranded nucleic acid.

4. The kit of claim 2 or 3, wherein the said fluorescent pigment is water-soluble and thermally stable at temperatures at which the said PCR is conducted.

5. The it of any one of claims 2 - 4 wherein the said vessel is provided with sealing means.

## Patentansprüche

1. Verfahren zum Nachweis einer Zielnucleinsäure in einer Probe, umfassend:
Amplifikation eines Bereiches der Zielnucleinsäure; Inkontaktbringen oder Behandlung des amplifizierten Bereiches mit einem Fluoreszenzfarbstoff, dessen Fluoreszenzeigenschaft sich durch die Reaktion mit der Nucleinsäure ändert; und
Nachweis oder Messen der Änderung in der Fluoreszenz des Markers, dadurch gekennzeichnet, daß der Fluoreszenzfarbstoff zu der Originalprobe zugesetzt wird und sich in einem Reaktionsgefäß befindet und die Amplifikation und der Nachweis oder das Messen der Fluoreszenz in dem Reaktionsgefäß in Gegenwart des Fluoreszenzfarbstoffs durchgeführt wird, und daß die Amplifikation mit Hilfe der Polymerasekettenreaktion durchgeführt wird.

2. Kit zum Nachweis oder der Quantifizierung einer Zielnucleinsäure in einer Probe, umfassend:
(A) einen Fluoreszenzfarbstoff, dessen Fluoreszenz sich durch die Reaktion mit einer doppelsträngigen Nucleinsäure ändert; und
(B) Reagenzien für die Amplifikation der Zielnucleinsäure durch die Polymerasekettenreaktion (PCR),
wobei der Fluoreszenzfarbstoff und die Reagenzien in einem einzigen Gefäß enthalten sind.

3. Kit nach Anspruch 2, wobei der Fluoreszenzfarbstoff ein Farbstoff ist, dessen Fluoreszenz sich durch Einlagerung in die doppelsträngige Nucleinsäure ändert.

4. Kit nach Anspruch 2 oder 3, wobei der Fluoreszenzfarbstoff wasserlöslich und bei den Temperaturen, bei denen die PCR durchgeführt wird, hitzestabil ist.

5. Kit nach einem der Ansprüche 2 bis 4, wobei das Gefäß mit einem Mittel zum Verschließen bereitgestellt ist.

## Revendications

1. Procédé pour la détection d'un acide nucléique cible dans un échantillon, comprenant :
l'amplification d'une région dudit acide nucléique cible;
la mise en contact ou le traitement de la région amplifiée avec un pigment fluorescent dont la propriété de fluorescence est modifiée par une réaction avec l'acide nucléique; et
la détection ou la mesure du changement de ladite fluorescence dudit marqueur, caractérisé en ce que le pigment fluorescent est ajouté à l'échantillon d'origine et situé dans un récipient de réaction et l'amplification et la détection ou la mesure de la fluorescence est effectuée dans le récipient de réaction en présence du pigment fluorescent et en ce que l'amplification est effectuée par une réaction à chaîne polymérase.

2. Trousse pour la détection ou la détermination quantitative d'un acide nucléique cible dans un échantillon, comprenant :
(A) un pigment fluorescent dont la fluorescence change lors d'une réaction avec un acide nucléique à double brins; et
(B) des réactifs pour l'amplification dudit acide nucléique cible par la réaction à chaîne polymérase (RCP),
dans laquelle ledit pigment fluorescent et lesdits réactifs sont contenus dans un seul récipient.

3. Trousse selon la revendication 2, dans laquelle ledit pigment fluorescent est un pigment dont la fluorescence change lors d'une insertion dans ledit acide nucléique à double brins.

4. Trousse selon la revendication 2 ou 3, dans laquelle ledit pigment fluorescent est soluble dans l'eau et stable thermiquement aux températures auxquelles ladite RCP est effectuée.

5. Trousse selon l'une quelconque des revendications 2-4, dans laquelle ledit récipient est muni d'un moyen de fermeture étanche.
